# EUROPEAN PATENT SPECIFICATION

(11) **EP 1 693 019 B1**
(45) Date of publication and mention of the grant of the patent: **10.10.2007**
(21) Application number: 06110141.6
(22) Date of filing: 20.02.2006
(51) Int. Cl.: A61B 19/02, B65D 77/04

(54) **Container for collecting and transporting special waste**
Behälter zum Sammeln und Transportieren von Sondermüll
Réceptacle collecteur et de transport de déchets spéciaux

(30) Priority: 21.02.2005 NL 1028352; 13.05.2005 NL 1029039; 08.09.2005 NL 1029903
(43) Date of publication of application: 23.08.2006
(73) Proprietor: Ecama Holding B.V., 3862 WL Nijkerk (NL)
(72) Inventor: Versluis, Evert, Cornelis, Antonie, 3862 WL, NIJKERK (NL)
(74) Representative: de Vries, Johannes Hendrik Fokke

(56) References cited:
- EP-A- 1 472 987
- US-A- 2 357 155
- US-A- 4 886 164
- US-A- 5 687 839
- US-B1- 6 283 909
- US-B1- 6 474 472

## Description

The invention relates to a container for collecting and transporting special waste, such as hospital waste and diagnostic samples according to the preamble of claim 1.

Such a container is known from EP-A-1 472 987, for example. It has been found from experiments with this known container that improvement is possible as regards the closure of the container. Furthermore, the closing edge of said known container is relatively complicated to manufacture.

The object of the invention is to provide an improved container of this kind.

To this end, according to the invention, a container is provided with the features of claim 1.

In this way a container is obtained in which a very strong bond between the upper edges of the inner box and the outer box and the adhesive strip in the cover is realised. In this way a hermetic seal of the container is obtained in the situation in which the cover is pressed onto the container. In addition, the upper edge of the outer box or the inner box that is covered by the plastic bag is easy to manufacture.

The invention will now be explained in more detail with reference to the drawing, which shows in a very schematic way some embodiments of the container according to the invention.
Fig. 1 schematically shows an embodiment of the container according to the invention, in which the inner box with the plastic bag is still positioned outside the outer box and the cover is not shown.
Fig. 2 is a perspective view of the cover of the container of Fig. 1.
Fig. 3 shows the container of Fig. 1, in which the inner box and the plastic bag are entirely contained within the outer box and the cover is pressed onto the container.
Fig. 4 is a sectional view along the line IV-IV in Fig. 3.
Fig. 5 is a sectional view corresponding to Fig. 4, in which a different embodiment of the cover is used.
Fig. 6 shows a second embodiment of the container according to the invention, in which a second inner box is used and the two inner boxes are still positioned outside the outer box.
Fig. 7 shows a box that forms part of an alternative embodiment of the container according to the invention.
Fig. 8 is a cross-sectional view of a variant of the cover of an embodiment of the container according to the invention.
Fig. 9 is a perspective view of an embodiment of the cover of a third embodiment of the container according to the invention.
Fig. 10 is a larger-scale view of a part of the cover of Fig. 9.
Fig. 11 is a perspective view from the bottom side of the cover of Fig. 9, with the peripheral edge of the cover in a temporary supported position.
Fig. 12 is a view along the line XII-XII of Fig. 11.
Fig. 13 is a sectional view corresponding to Fig. 4 of the third embodiment of the container according to the invention, in which the cover of Fig. 11 is placed on the outer box in the temporary supported position.

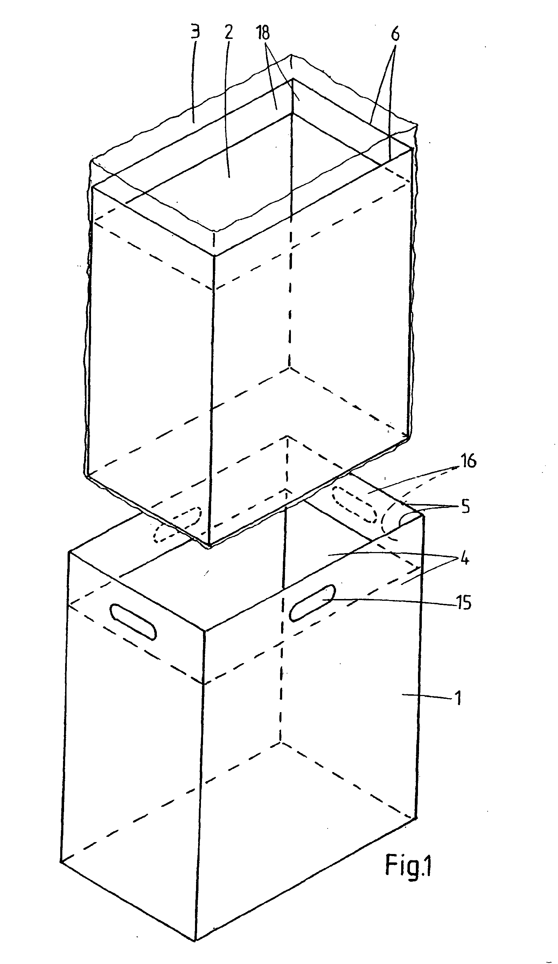

Figs. 1-3 show an embodiment of the container for collecting and transporting special waste. This container comprises an outer box 1 and an inner box 2, which is still positioned outside the outer box in the view that is shown in Fig. 1. Present between the outer box 1 and the inner box 2 is a plastic bag 3, which is made of so-called shrink foil, for example biaxially shrinkable LDPE having a thickness of 60-70 µ. The outer box 1 and the inner box 2 have a bottom with a so-called American closure, with this understanding that the bottom flaps formed on the side walls 4 partially overlap each other, so that no openings are present in the bottom in the assembled state of the box. The plastic bag 3 likewise has a closed bottom. When the inner box 2 is placed in the plastic bag 3, the plastic bag 3 projects about four cm above the inner box.

The outer box 1 and the inner box 2 of the container as described consist of a water resistant, solid cardboard, in particular a solid, water resistant glued cardboard with a plastic coating of polyethylene, for example. Preferably, at least 600 g/m2 cardboard is used. The thickness of the material of the solid cardboard of the outer box 1 and of the inner box 2 preferably meets the standards as regards resistance to puncture by sharp objects of, for example, British Standard BS7320 (1990).

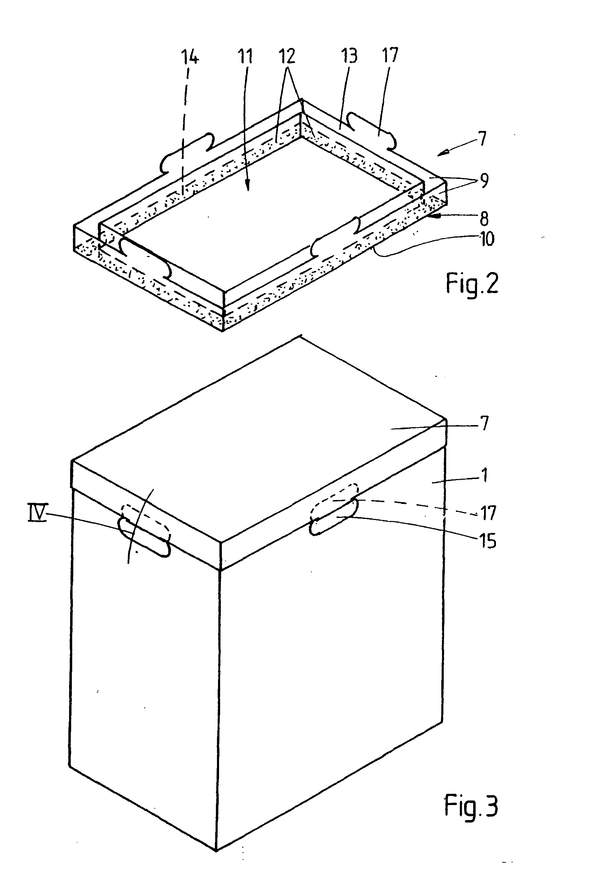

Upon manufacture of the container, the inner box 2 is placed into the plastic bag 3 and the inner box 2 with the plastic bag is inserted into the outer box 1. The dimensions of the outer box 1 and the inner box 2 have been selected so that when the inner box 2 rests on the bottom of the outer box 1, the upper edges 5 and 6 of the outer box 1 and the inner box 2 lie at least substantially in the same plane. Then the projecting part of about 4 cm of the plastic bag 3 is folded down outwards and the container is subjected to a heat treatment. As a result, the part of the plastic bag 3 that abuts against the upper part of the outer wall of the outer box 1 contracts tightly around the outer wall of the outer box 1. In practice the material of the plastic bag 3 will also bond firmly to the plastic coating of the outer box 1, as a result of which a smoothly finished surface is obtained in particular at the upper edge 5 so as to provide a watertight and airtight seal of the container by means of a cover 7 as shown in Fig. 2.

In the illustrated embodiment, the cover 7 comprises an outer cover 8 with a peripheral edge 9. Said peripheral edge 9 is in one piece with the upper surface 10 of the outer cover 8 and has been formed together with the upper surface 10 of a blank without any cuts. An inner cover 11 is fitted in the outer cover 8, so that a channel 13 is formed between the peripheral edge 9 and an inner edge 12 of the inner cover 11. An adhesive strip 14 is provided in said channel 13, which adhesive strip preferably consist of a flexible hot melt adhesive, for example a hot melt with a base of a synthetic polymer. Said hot melt adhesive may or may not be foamed. The cover 7 is made of the same material as the outer box 1 and the inner box 2.

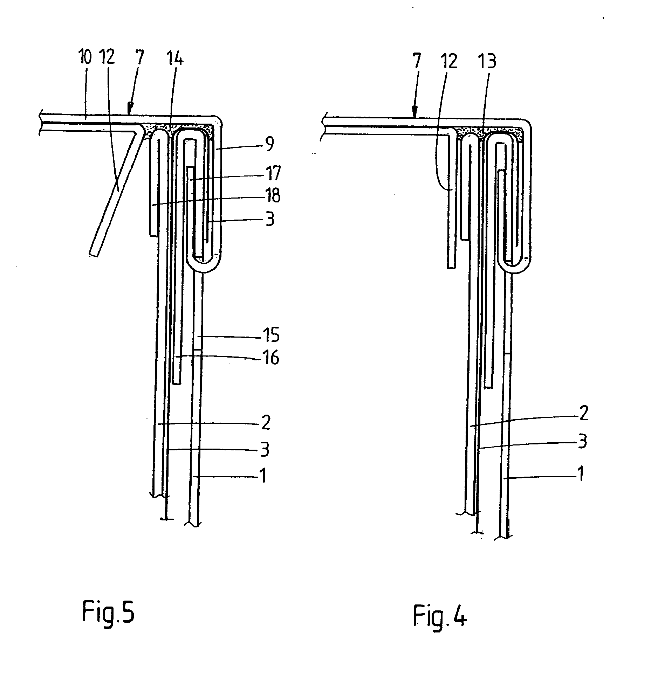

In Fig. 3 the cover 7 is pressed onto the outer box 1 with the inner box 2 and the plastic bag 3 present therein. In this closure position of the cover 7, the peripheral edge 9 encloses the upper part of the outer box 1 and the upper edges 5 and 6 of the outer box 1 and the inner box 2 respectively are pressed into the adhesive strip 14, as is shown in the sectional view of Fig. 4. Tests have shown that in this way a fully hermetic seal of the container is obtained. The sealed container meets all the standards that are made of containers for hospital waste.

As is shown in Fig. 1, each side wall 4 of the outer box 1 is provided with a handle opening 15, and top flaps 16 are provided on the side walls 4, which flaps are folded against the inner side of the side walls 4. In this way the handle openings 15 are covered, so that the contents of the container are protected by a double layer of cardboard also at the location of the handle openings 15. The handle openings 15 are not only intended for lifting the container, but they also function as connecting means in order to ensure that the cover 7 is pressed onto the container in the correct closure position. The peripheral edge 9 of the cover 7 is provided with connecting lips 17, which can be folded into the handle openings 15 when the cover 7 is fully pressed onto the container. The connecting lips 17 are retained between the side walls 4 and the top flaps 16 in that case.

According to an alternative embodiment, the outer box 1 may not be provided with the top flaps 16 or with top flaps that do not cover the handle openings. The connecting lips 17 are retained between the side walls of the outer box 1 and the side walls of the inner box 2 in that case. The advantage of retaining the connecting lips between two box parts, i.e. the top flaps and the side walls of the outer box and the side walls of the outer box and the inner box, respectively, in the variants described herein is that in case of an excess pressure in the container relative to the environment the connecting lips will be wedged against the side walls of the outer box 1, with the wedging force increasing as the excess pressure increases. It is also possible, of course, not to configure the openings 15 as handle openings, in which case the openings only function as locking openings for receiving the connecting lips 17. It is noted that the locking engagement of the cover 7 on the container as described can also be used with containers of a type different from that of the containers that are described herein. The container may comprise an inner box, an outer box and a cover made of different materials.

It is noted that it will suffice, if desired, to provide two opposite side walls 4 and parts of the peripheral edge 9 of the cover 7 with handle openings 15 and connecting lips 17 respectively. Furthermore it is possible to use two or more handle openings and connecting lips on one side.

Fig. 5 is a view corresponding to Fig. 4 of a container according to the invention, in which an alternative embodiment of the cover 7 is used. In this case the inner cover 11 is provided with a sloping inner edge 12, which slope enables the inner edge 12 to function as a guide edge for the upper edge 6 of the inner box 2. In this way it is ensured that the upper edge 6 of the inner box 2 is pressed into the adhesive strip 14 over the entire circumference thereof.

As is shown schematically in Fig. 1, the side walls of the inner box 2 are provided with top flaps 18 as well, which top flaps are folded against the inner side of the side walls, so that a smooth, strong upper edge 6 of the inner box 2 is obtained. Although the upper edge 6 of the inner box 2 is not covered by a plastic bag 3, the plastic coating of the material of the inner box provides a smoothly finished upper edge 6, which helps to obtain a good hermetic seal by means of the cover 7.

Alternatively, the projecting part of the plastic bag 3 can also be folded down inwards, so that the plastic material will come to lie against the inner side of the wall of the inner box 2. After the heat treatment a smoothly finished surface of the upper edge 6 of the inner box is obtained. In this variant of the inner box 2, the heat treatment can be carried out before the top flaps 18 are folded inwards. As a result, the plastic bag is pulled tightly over the top flaps 18 and the plastic material will adhere to the coating of the top flaps 18. After the heat treatment, the upper flaps 18 are folded inwards together with the plastic material of the plastic bag 3 adhering thereto, as a result of which the plastic material is pulled tightly over the upper edge 6 of the inner box 2.

The part of the plastic bag 3 present between the two boxes 1 and 2 is more or less freely positioned between the two boxes. As a result, air is retained within the plastic bag 3 when the container is closed with the cover 7, which air has a damping effect when the container is subjected to shock loads. The airtight closure of the plastic bag 3 is ensured in that the plastic bag encloses the upper edge 5 of the outer box 1 or the upper edge 6 of the inner box 2 and is pressed into the adhesive strip 14 when the cover 7 is placed on the container.

It is noted that the plastic bag 3 may also consist of a plastic material other than a shrink foil. In that case the upper end of the plastic bag 3 can be folded over the top flaps 18 of the inner box 2, for example, and be folded inwards together with the top flaps 18, as a result of which the plastic material is pulled tightly around the upper edge 6 of the inner box.

An amount of absorption material may be present in the container, if desired, which material is capable of absorbing any moisture that may be present in the waste. The dimensions (1 x w x h) of the outer box are 40 x 30 x 50 cm, for example.

An advantage of the container as described herein is that a hermetic seal of the outer box 1 and the inner box 2 by the cover 7 can be effected by using relatively simple means. The container meets the usual standards as regards puncture resistance and falling resistance for containers of hospital waste. Another advantage of the container as described herein is that when more stringent requirements are to be met, this can be done by using a heavier type of cardboard. The cardboard container as described herein furthermore has this advantage that the dimensions can readily be changed, without any costly investments in new moulds being required, as is the case with containers of plastic material.

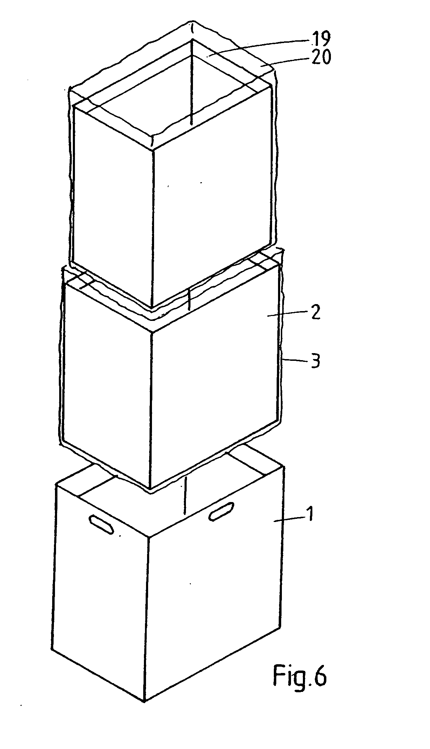

The container can readily be extended in a modular fashion in those cases in which very stringent requirements are to be met, for example when UN packing instruction P620 applies. A possible variant is shown in Fig. 6, in which a second inner box 19 is used. Said inner box 19 is placed into a plastic bag 20 and is inserted into the inner box 2 together with said plastic bag 20, after which the plastic bag is folded over the side walls of the inner box 2 and pulled tightly around the upper side of the inner box 2 as a result of being subjected to a heat treatment. Then the inner boxes 2 and 19 are placed into the outer box 1 together with the plastic bag 3 and the plastic bag 3 is pulled tightly around the upper side of the outer box 1 in the above-described manner. One or more additional inner boxes 19 may be used, depending on the requirements that the container is to meet.

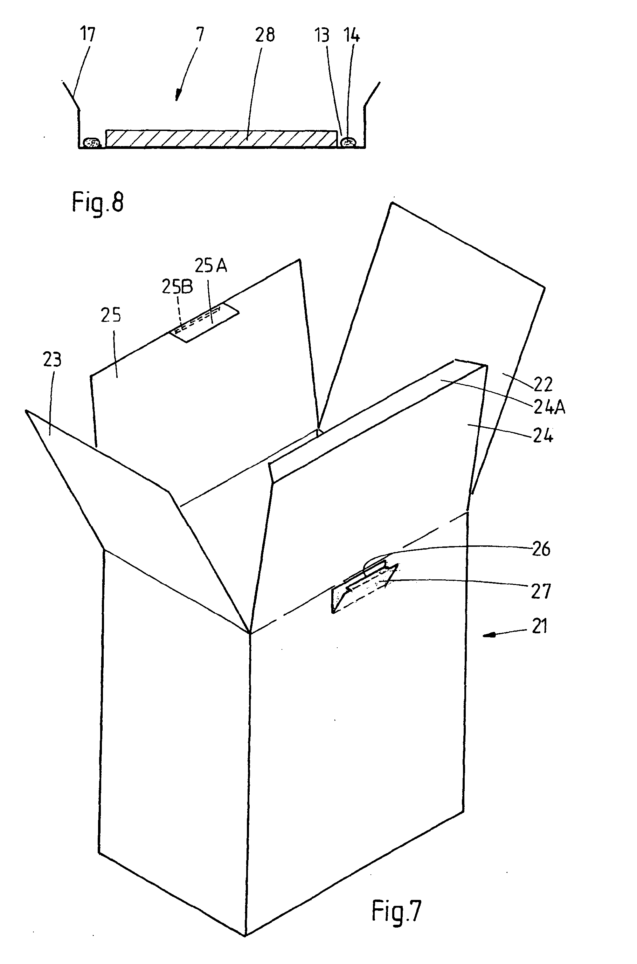

Fig. 7 shows an embodiment of a box 21 capable of accommodating the outer box 1 and the inner box 2. It is furthermore possible to place assemblies of two or more assembled outer and inner boxes 1,2 into said box 21. The box 21 is provided with two top flaps 22 and 23, which are arranged one on top of the other so as to close the box 21. Then a top flap 24 comprising a closure flap 24a is laid over the top flaps 22,23, with the closure flap 24a being inserted between the side wall of the box 21 and the top flaps 22,23. Finally, a top flap 25 provided with a closure lip 25a is laid over the top flap 24 and the closure lip is inserted into the opening 26. A slot 25b is formed in the closure lip 25a, into which slot a closure lip 27 is inserted.

Fig. 8 shows an alternative embodiment of the cover 7, which is made up of the outer cover 8 and a plate 28 of a plastic material, for example polystyrene, provided therein. Between the plate 28 and the peripheral edge 9 the channel 13 is formed, in which the adhesive strip 14 is provided. The plate 28 may comprise a sloping locating edge for the upper edge 6 of the inner box 2. If desired, a cover 7 not provided with an inner cover 11 or a plate 28 can be used.

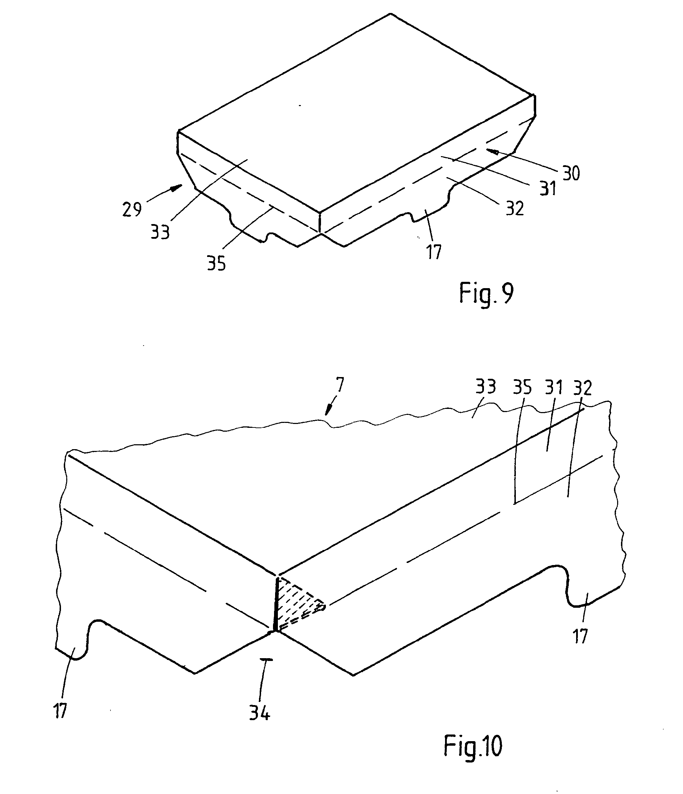

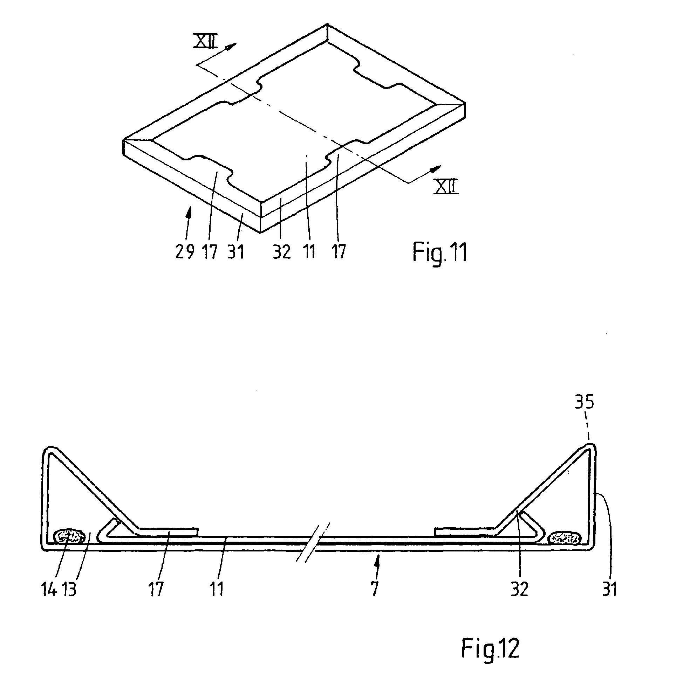

Figs. 9-12 show an advantageous embodiment of a cover 29 for a container according to the invention, in which the outer box 1 and the inner box 2 of Fig. 1 can be used. The cover 29 is configured with a peripheral edge 30 similarly to the cover 7 of Fig. 2, which edge in this case consists of two parts 31 and 32, of which the peripheral edge part 31 joins the upper panel 33 and is completely closed in the corners, as is shown schematically in Fig. 10. The peripheral edge part 32 is open in the corners, as indicated at 34, so that the peripheral edge part 32 can be folded inwards about the fold lines indicated by a dashed line 35 to the position that is shown in Figs. 11 and 12. The peripheral edge part 32 is maintained in this temporary position of use by the connecting lips 17 on the inner cover 11 or, if an inner cover is not used, by temporarily affixing it to the inner side of the upper panel 33, for example by means of a suitable adhesive. In this way the adhesive strip 14 in the channel 13 is covered by the peripheral edge portion 32, so that the adhesive strip 14 is fully protected. The peripheral edge part 32 functions as a covering edge for the channel 13 and the adhesive strip 14, as it were.

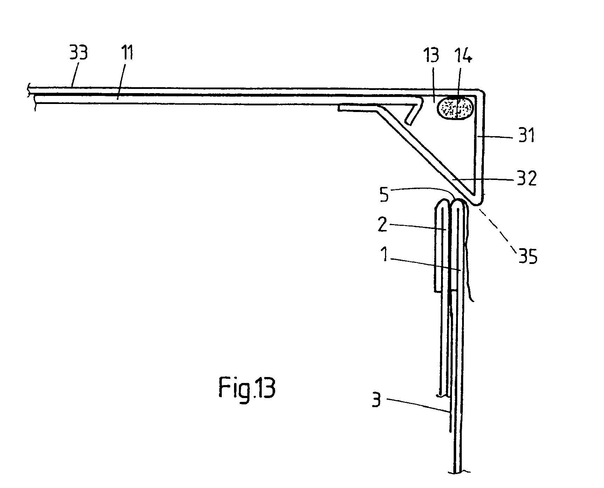

In addition, the cover 29 can easily be placed in a temporary supported position on the outer box 1 and the inner box 2, as is shown in sectional view in Fig. 13. In this position, the cover 29 engages the upper edge 5 of the outer box 1 with the peripheral edge part 32, as a result of which a more or less airtight temporary closure of the container is obtained. Once the inner box 2 has been filled, the connecting lips 17 are pulled off the inner side of the upper panel 33 or the inner cover 11, and the cover 29 is placed permanently on the outer box 1 and the inner box 2, with the upper edges 5,6 of the boxes 1,2 being pressed into the adhesive strip 14 and the connecting lips 17 being folded into the handle openings 15.

For the sake of completeness it is noted that in those cases in which the cover 7 or 29 is not provided with an inner cover, this single cover may be made of a heavier type of cardboard so as to obtain the required puncture resistance.

According to another variant of the container according to the invention, it is also possible to place a container of a plastic material provided with a cover of its own into the container that is shown in Fig. 1, which may be extended with one or more inner boxes 19. Furthermore, one or more so-called needle boxes may be placed in the container according to the invention. The needle boxes may be formed in a relatively simple manner of a puncture resistant cardboard (for example according to British Standard BS7320).

Although the invention has been described herein by means of embodiments of containers for collecting and transporting special waste, the containers described may also be used for collecting and transporting other materials. Another application is the packing and shipping of diagnostic samples.

The invention is not restricted to the embodiments described above, which can be varied in several ways without departing from the scope of the claims.

## Claims

1. A container for collecting and transporting special waste, such as hospital waste and diagnostic samples, which container comprises an outer box (1) of cardboard, an inner box (2) of cardboard, which can be placed in the outer box, a plastic bag (3) positioned between the outer box and the inner box and fold inwards over the upper edge (6) of the inner box, which outer box and inner box each have a closed bottom and an open upper side, and a cover (7,29) that can be pressed onto the container in a closure position for closing the container, **characterized in that** the plastic bag (3) is folded outwards over the upper edge (5) of the outer box (1) or inwards over the upper edge (6) of the inner box (2) and lies against the upper part of the wall of the outer box or the inner box, respectively, with the cover (7,29) having a peripheral edge (9,31), which encloses the upper part of the outer wall of the outer box in the closure position and which is provided with an adhesive strip (14) on the inner side, in which the upper edges of the outer box and the inner box are accommodated when the cover is pressed on the container in the closure position.

2. A container according to claim 1, wherein the outer box (1) and the inner box (2) and the cover (7,29) are made of a water-resistant, solid cardboard, preferably with a coating of polyethylene.

3. A container according to claim 1 or 2, wherein the cover (7,29) is provided with a channel (13) on the inner side of the peripheral edge (9,31), the outer side of which channel is bounded by the peripheral edge and the inner side is bounded by an inner edge (12), in which channel the adhesive strip (14) is present.

4. A container according to claim 3, wherein the inner edge (12) of the channel (13) is configured as a guide edge for the upper edge (6) of the inner box (2).

5. A container according to any one of the preceding claims, wherein the cover (29) is provided with a cover edge (32) joining the peripheral edge (31), which covers the adhesive strip (14), which cover edge is movable between a supported position, in which the cover can be placed on the upper edge (5) of the outer box (1) in such a manner as to be removable therefrom, and an operative position, in which the cover can be pressed onto the container in the closure position.

6. A container according to claim 5, wherein the cover edge (32) is in one piece with the peripheral edge (31), wherein the cover edge is aligned with the peripheral edge in the operative position and extends between the peripheral edge and the inner side of the cover in the supported position.

7. A container according to any one of the preceding claims, wherein the outer box (1) is provided with a handle opening (15) in at least two opposite side walls (4) and with a top flap (16) that lies against the inner side of the outer wall, thereby covering the handle opening.

8. A container according to any one of the preceding claims, wherein the cover (7,29) and the outer box (1) are provided with interacting connecting means (15, 17) which can be interconnected in the closure position of the cover.

9. A container according to claim 7 and 8, wherein the handle openings (15) form the connecting means of the outer box (1) and wherein the peripheral edge (9,31) or the cover edge (32) of the cover (29) is provided with connecting lips (17) which can be accommodated in the handle openings (15) in the closure position.

10. A container according to claim 7, 8 and 9, wherein all four side walls (4) of the outer box (1) are provided with a handle opening (15) and a top flap (16), which top flaps are folded inwards, thereby covering the associated handle opening, wherein the peripheral edge (9) or the cover edge (32) of the cover (7, 29) is provided with a connecting lip (17) for each handle opening, which connecting lip (17) can be accommodated in the associated handle opening (15) in the closure position.

11. A container according to any one of the preceding claims, wherein the inner box (2) is provided with top flaps (18) that lie against the inner side of the outer wall.

12. A container according to any one of the preceding claims, wherein the upper part of the plastic bag (3) abuts tightly against the wall of the outer box or the inner box, whilst the remaining part of the plastic bag is contained between the outer box (1) and the inner box (2) with some play.

13. A container according to any one of the preceding claims, wherein a second plastic bag is present between the outer box (1) and the inner box (2), wherein the first plastic bag (3) is folded inwards over the upper edge (6) of the inner box and wherein the second plastic bag is folded down outwards over the upper edge (5) of the outer box.

14. A container according to any one of the preceding claims, comprising at least one second inner box (19) and a second plastic bag (20) which is folded over the upper edge of the first (2) or the second (19) inner box and which abuts tightly against the upper part of the wall of said inner box.

15. A container according to any one of the preceding claims, comprising an inner container of plastic material provided with a cover of plastic material.

16. A container according to any one of the preceding claims, wherein at least the folded part of the plastic bag (3) tightly encloses the upper edge and the upper part of the wall of the outer box (1) or the inner box (2).

## Patentansprüche

1. Behälter zum Sammeln und Transportieren von Spezialabfällen, wie Krankenhausabfälle und diagnostischer Proben, welcher Behälter umfasst eine äußere Box (1) aus Pappe, eine innere Box (2) aus Pappe, welche in der äußeren Box platziert werden kann, eine Plastiktasche (3), welche zwischen der äußeren und der inneren Box platziert ist, und über die obere Kante (6) der inneren Box eingeschlagen ist, wobei die äußere Box und die innere Box jeweils einen geschlossenen Boden und eine offene obere Seite aufweisen, und eine Abdeckung (7, 29) nach innen, welche in einer geschlossenen Position auf den Behälter gepresst werden kann, um den Behälter zu verschließen, **dadurch gekennzeichnet, dass** die Plastiktasche (3) über die obere Kante (5) der äußeren Box (1) nach außen oder über die obere Kante (6) der inneren Box (2) gefaltet ist und am oberen Teil der Wand der äußeren Box bzw. der inneren Box anliegt, wobei die Abdeckung (7, 29) eine Umfangskante aufweist (9, 31), welche den oberen Teil der äußeren Wand der äußeren Box in der geschlossenen Position einschließt und welche auf der inneren Seite mit einem Haftstreifen (14) versehen ist, in welchem die oberen Kanten der äußeren Box und der inneren Box aufgenommen sind, wenn die Abdeckung in der geschlossenen Position auf den Behälter gedrückt ist.

2. Behälter gemäß Anspruch 1, wobei die äußere Box (1) und die innere Box (2) und die Abdeckung (7, 29) aus einem Wasser beständigen Material, einer festen Pappe, vorzugsweise mit einer Beschichtung aus Polyethylen, gemacht sind.

3. Behälter gemäß Anspruch 1 oder 2, wobei die Abdeckung (7, 29) mit einem Rille (13) auf der inneren Seite der Umfangskante (9, 31) ausgestattet ist, die äußere Seite dieser Rille durch die Umfangskante eingeschlossen ist und die innere Seite durch eine innere Kante (12) eingeschlossen ist, wobei sich in dieser Rille der Haftstreifen (14) befindet.

4. Behälter gemäß Anspruch 3, wobei die innere Kante (12) der Rille (13) als eine Richtkante für die obere Ecke (6) der inneren Box (2) gestaltet ist.

5. Behälter gemäß einem der zuvor genannten Ansprüche, wobei die Abdeckung (29) mit einer Abdeckkante ausgestattet ist (32), welche die Umfangskante (31) trifft, welche den Klebestreifen (14) abdeckt, wobei jene Abdeckkante beweglich ist zwischen einer gestützten Position, in welcher die Abdeckung auf der oberen Kante (5) der äußeren Box so platziert werden kann, dass sie von dort entfernbar ist, und einer Betriebsposition, in welcher die Abdeckung auf den Behälter in die geschlossene Position gepresst werden kann.

6. Behälter gemäß Anspruch 5, wobei die Abdeckkante (32) in einem Stück mit der Umfangskante (31) ist, wobei die Abdeckkante mit der Umfangskante in der Betriebsposition fluchtet und in der gestützten Position sich zwischen der Umfangskante und der inneren Seite der Abdeckung erstreckt.

7. Behälter gemäß einem der zuvor genannten Ansprüche, wobei die äußere Box (1) mit einer Handgrifföffnung (15) in mindestens zwei gegenüberliegenden Seitenwänden (4) ausgestattet ist sowie mit einer oberen Klappe (16), welche gegen die obere Seite der äußeren Wand anliegt, und dabei die Handgrifföffnung abdeckt.

8. Behälter gemäß einem der zuvor genannten Ansprüche, wobei die Abdeckung (7, 29) und die äußere Box (1) mit interagierenden Verbindungselementen (15, 17) ausgestattet sind, welche in der Verschlussposition der Abdeckung miteinander verbunden werden können.

9. Behälter gemäß den Ansprüchen 7 und 8, wobei die Handgrifföffnungen (15) die Verbindungselemente der äußeren Box (1) bilden und wobei die Umfangskante (9, 31) oder die Abdeckkante (32) der Abdeckung (29) mit verbindenden Lippen (17) ausgestattet sind, welche in den Handgrifföffnungen (15) in der geschlossenen Position aufgenommen werden können.

10. Behälter gemäß den Ansprüchen 7, 8 und 9, wobei alle vier Seitenwände (4) der äußeren Box (1) mit einer Handgrifföffnung (15) und einer oberen Klappe (16) ausgestattet sind, wobei diese oberen Klappen nach innen gefaltet sind, und dabei die entsprechenden Handgrifföffner abdecken, wobei die Umfangskante (9) oder die Abdeckkante (32) der Abdeckung (7, 29) mit Verbindungslippen (17) für jede Handgrifföffnung ausgestattet sind, wobei diese Verbindungslippen (17) im entsprechenden Handgrifföffner (15) in der geschlossenen Position aufgenommen werden können.

11. Behälter gemäß einem der zuvor genannten Ansprüche, wobei die innere Box (2) mit oberen Klappen (18), die gegen die innere Seite der äußeren Wand liegen, versehen ist.

12. Behälter gemäß einem der zuvor genannten Ansprüche, wobei der obere Teil der Plastiktasche (3) gegen die Wand der äußeren Box oder der inneren Box eng anliegt, während der restliche Teil der Plastiktasche zwischen der äußeren Box (1) und der inneren Box (2) mit einem gewissen Spiel aufgenommen wird.

13. Behälter gemäß einem der zuvor genannten Ansprüche, wobei eine zweite Plastiktasche zwischen der äußeren Box (1) und der inneren Box (2) existiert, wobei die erste Plastiktasche (3) nach innen über die obere Ecke (6) der inneren Box gefaltet ist und wobei die zweite Plastiktasche nach außen über die obere Kante (5) der äußeren Box nach unten gefaltet ist.

14. Behälter gemäß einem der zuvor genannten Ansprüche, welcher mindestens eine zweite innere Box (19) und eine zweite Plastiktasche (20) aufweist, welche über die obere Ecke der ersten (2) oder der zweiten (19) inneren Box gefaltet ist und welche gegen den oberen Teil der Wand der inneren Box eng anliegt.

15. Behälter gemäß einem der zuvor genannten Ansprüche, mit einem inneren Behälter aus Plastikmaterial, welcher mit einer Abdeckung aus Plastikmaterial ausgestattet ist.

16. Behälter gemäß einem der zuvor genannten Ansprüche, wobei mindestens der gefaltete Teil der Plastiktasche (3) die obere Kante und den oberen Teil der Wand der äußeren Box (1) oder der inneren Box (2) eng umschließt.

## Revendications

1. Réceptacle de collecte et transport de déchets spéciaux, tels que les déchets hospitaliers et les échantillons de diagnostique, lequel réceptacle comprend une boîte externe (1) en carton, une boîte interne (2) en carton, qui peut être placée dans la boîte externe, un sac plastique (3) positionné entre la boîte externe et la boîte interne et plié vers l'intérieur au-dessus du bord supérieur (6) de la boîte interne, lesquelles boîte externe et boîte interne présentent chacune un fond fermé et un côté supérieur ouvert, et un couvercle (7, 29) qui peut être pressé sur le réceptacle dans une position de fermeture destinée à fermer le réceptacle, **caractérisé en ce que** le sac plastique (3) est plié vers l'extérieur au-dessus du bord supérieur (5) de la boîte externe (1) ou vers l'intérieur au-dessus du bord supérieur (6) de la boîte interne (2) et se situe contre la partie supérieure de la paroi de la boîte externe ou de la boîte interne, respectivement, avec le couvercle (7, 29) présentant un bord périphérique (9, 31), qui enferme la partie supérieure de la paroi externe de la boîte externe dans la position de fermeture et qui est pourvu d'une bande adhésive (14) sur le côté interne, dans lequel sont logés les bords supérieurs de la boîte externe et de la boîte interne lorsque le couvercle est pressé sur le réceptacle dans la position de fermeture.

2. Réceptacle selon la revendication 1, dans lequel la boîte externe (1) et la boîte interne (2) et le couvercle (7, 29) sont fabriqués à partir d'un carton solide résistant à l'eau, revêtu de préférence de polyéthylène.

3. Réceptacle selon la revendication 1 ou 2, dans lequel le couvercle (7, 24) est pourvu d'un canal (13) sur le côté interne du bord périphérique (9, 31), canal dont le côté externe est délimité par le bord périphérique et dont le côté interne est délimité par un bord périphérique (12), canal dans lequel la bande adhésive (14) est présente.

4. Réceptacle selon la revendication 3, dans lequel le bord interne (12) du canal (13) est configuré comme bord de guidage pour le bord supérieur (6) de la boîte interne (2).

5. Réceptacle selon l'une quelconque des revendications précédentes, dans lequel le couvercle (29) est pourvu d'un bord de couvercle (32) reliant le bord périphérique (31), qui recouvre la bande adhésive (14), lequel bord de couvercle est mobile entre une position supportée, dans laquelle le couvercle peut être placé sur le bord supérieur (5) de la boîte externe (1) de manière à pouvoir en être retiré, et une position opérationnelle, dans laquelle le couvercle peut être pressé sur le réceptacle dans la position de fermeture.

6. Réceptacle selon la revendication 5, dans lequel le bord de couvercle (32) ne fait qu'un avec le bord périphérique (31), dans lequel le bord du couvercle est aligné sur le bord périphérique dans la position opérationnelle et s'étend entre le bord périphérique et le côté interne du couvercle dans la position supportée.

7. Réceptacle selon l'une quelconque des revendications précédentes, dans lequel la boîte externe (1) est pourvue d'une ouverture de poignée (15) dans au moins deux parois latérales opposées (4) et d'un rabat supérieur (16) qui se situe contre le côté interne de la paroi externe, recouvrant de ce fait l'ouverture de poignée.

8. Réceptacle selon l'une quelconque des revendications précédentes, dans lequel le couvercle (7, 29) et la boîte externe (1) sont pourvus de moyens de raccordement en interaction (15, 17) qui peuvent être interconnectés dans la position de fermeture du couvercle.

9. Réceptacle selon les revendications 7 et 8, dans lequel les ouvertures de poignée (15) forment les moyens de raccordement de la boîte externe (1) et dans lequel le bord périphérique (9, 31) du bord de couvercle (32) du couvercle (29) est pourvu de lèvres de raccordement (17) qui peuvent être logées dans les ouvertures de poignée (15) dans la position de fermeture.

10. Réceptacle selon les revendications 7, 8 et 9, dans lequel les quatre parois latérales (4) de la boîte externe (1) sont toutes pourvues d'une ouverture de poignée (15) et d'un rabat supérieur (16), lesquels rabats supérieurs sont pliés vers l'intérieur, recouvrant de ce fait l'ouverture de poignée associée, dans lequel le bord périphérique (9) ou le bord de couvercle (32) du couvercle (7, 29) est pourvu d'une lèvre de raccordement (17) pour chaque ouverture de poignée, laquelle lèvre de raccordement (17) peut être logée dans l'ouverture de poignée (15) associée dans la position de fermeture.

11. Réceptacle selon l'une quelconque des revendications précédentes, dans lequel la boîte interne (2) est pourvue de rabats supérieurs (18) qui se situent contre le côté interne de la paroi externe.

12. Réceptacle selon l'une quelconque des revendications précédentes, dans lequel la partie supérieure du sac plastique (3) est étroitement en butée contre la paroi de la boîte externe ou de la boîte interne, tandis que la partie restante du sac plastique est contenue entre la boîte externe (1) et la boîte interne (2) avec un certain jeu.

13. Réceptacle selon l'une quelconque des revendications précédentes, dans lequel un second sac plastique est présent entre la boîte externe (1) et la boîte interne (2), dans lequel le premier sac plastique (3) est plié vers l'intérieur au-dessus du bord supérieur (6) de la boîte interne et dans lequel le second sac plastique est plié vers le bas et l'extérieur au-dessus du bord supérieur (5) de la boîte externe.

14. Réceptacle selon l'une quelconque des revendications précédentes, comprenant au moins une seconde boîte interne (19) et un second sac plastique (20) qui est plié au-dessus du bord supérieur de la première (2) ou de la seconde (19) boîte interne et qui est étroitement en butée contre la partie supérieure de la paroi de ladite boîte interne.

15. Réceptacle selon l'une quelconque des revendications précédentes, comprenant un réceptacle interne en matière plastique pourvu d'un couvercle en matière plastique.

16. Réceptacle selon l'une quelconque des revendications précédentes, dans lequel au moins la partie pliée du sac plastique (3) entoure étroitement le bord supérieur et la partie supérieure de la paroi de la boîte externe (1) ou de la boîte interne (2).
